# EUROPEAN PATENT SPECIFICATION

(11) **EP 2 921 856 B1**
(45) Date of publication and mention of the grant of the patent: **14.09.2016**
(21) Application number: 15159467.8
(22) Date of filing: 17.03.2015
(51) Int. Cl.: G01N 33/53, A61K 47/48

(54) **A QUANTITATIVE ASSAY FOR 4-PYRROLIDINOPYRIDINE (4-PPY) IN POLYSACCHARIDE-PROTEIN CONJUGATE VACCINES**
QUANTITATIVER TEST FÜR 4-PYRROLIDINOPYRIDIN (4-PPY) IN KONJUGATIMPFSTOFFEN AUS POLYSACCHARID-PROTEIN
ANALYSE QUANTITATIVE POUR DE LA 4-PYRROLIDINOPYRIDINE (4-PPY) DANS DES VACCINS CONJUGUÉS POLYSSACHARIDE-PROTÉINE

(30) Priority: 18.03.2014 IN MU08922014
(43) Date of publication of application: 23.09.2015
(73) Proprietor: Serum Institute Of India Private Limited, Pune 411 028, Maharashtra (IN)
(72) Inventor: Patil, Kundan Dharma, 411028 Maharashtra (IN); Gairola, Sunil Jagdishprasad, 411028 Maharashtra (IN); Ashtagi, Siddharam Chandrakant, 411028 Maharashtra (IN); Avalskar, Nikhil Dattatray, 411028 Maharashtra (IN)
(74) Representative: Pons

(56) References cited:
- WO-A2-00/56360
- WO-A2-2011/084705
- US-A1- 2009 298 794
- US-B2- 8 273 789

## Description

Vaccines that contain protein covalently linked to carbohydrate have proven remarkably successful in inducing an immune response to the carbohydrate moiety. Examples of such vaccines, known as "conjugates" are available for Haemophilus influenzae type b (e.g., ActHib, Hiberix), Neisseria meningitidis types A C W and Y (e.g., Menactra) and S. pneumoniae (e.g., Prevnar, Synflorix). In order for the protein to be linked to the carbohydrate, the latter generally needs to be activated so that it can be reacted with the protein, either directly or via a spacer (Dick, W.E. Jr and Beurret, M. Glyco-conjugates of bacterial carbohydrate antigens. A survey and consideration of design and preparation factors. In: Conjugate Vaccines (Eds Cruse, J.M. and Lewis, R.E.).

Improved methods for cyanating polysaccharides use 1 -cyano-4-dimethylaminopyridine tetrafluoroborate (CDAP) (Lees, A., Producing immunogenic constructs using soluble carbohydrates activated via organic cyanylating reagents. U.S. Patent Nos. 5,651,971; 5,693,326; and 5,849,301). GSK's Synflorix is an approved multivalent pneumococcal polysaccharide-protein conjugate vaccine prepared by using CDAP based cyanylation conjugation chemistry.Further GSK's Nimenrix is a pipeline multivalent meningococcal polysaccharide-protein conjugate vaccine prepared by using CDAP based cyanylation conjugation chemistry. The activation of the polysaccharide with CDAP introduces a cyanate group in the polysaccharide and DMAP(4-dimethylamino-pyridin) is liberated. It is known that residual DMAP results in degradation of polysaccharide .Further DMAP has a rat LD50 (oral)of 140 mg/kg. To address above concerns of DMAP and subsequent queries raised by regulatory agencies , GSK has previously disclosed in-house assays to quantify residual DMAP content in CDAP conjugation chemistry based polysaccharide-protein conjugate vaccines (Synflorix, Nimenrix)as well as utilized diafiltration for removal of DMAP.Refer WO00/56359 and WO2007000342.

Regulatory authorities do consider potential toxicity of the identified exogenous process related impurities and accordingly require that the same should be considerably lower than the 1.5 µg/dose .Refer EMEA Guideline(EMEA/CHMP /QWP/251344/2006) on 'The Limits of Genotoxic Impurities'.

It has been reported previously that the polysaccharide-protein conjugate purification procedures should remove residual reagents used for conjugation and the removal of reagents and reaction by-products such as cyanide, EDAC and others depending on the conjugation chemistry, should be confirmed by suitable tests or by validation of the purification process wherein the acceptable limits should be approved by the NRA. Refer Section A.3.3.1 WHO/BS/06.2041 2006 "Recommendations to assure the quality, safety and efficacy of group A meningococcal conjugate vaccines"

Polysaccharide- protein conjugate vaccines prepared using 1-Cyano-4-pyrrolidinopyridinium tetrafluorborate (CPPT)based cyanylation conjugation chemistry are still in development phase and yet to be approved by regulatory agencies.Inventors have surprisingly found that during conjugation process 1-Cyano-4- pyrrolidinopyridinium tetrafluorborate gets hydrolysed to form a reaction by-product"4-Pyrrolidinopyridine (4-PPY)".

Further 4-PPY has a rat LD50 (oral) of 176 mg/kg.To address above concerns of 4-PPY and queries that could be raised by regulatory agencies , there is an urgent need to develop an assay to quantify residual 4-PPY content in CPPT conjugation chemistry based polysaccharide-protein conjugate vaccines.Particularly, quantification of 4-PPY would be necessary for pipeline multivalent meningococcal polysaccharide protein conjugate vaccine compositions comprising capsular saccharide from serogroups X and capsular saccharide from A, C, W135 and Y prepared by using 1-Cyano-4-pyrrolidinopyridinium tetrafluorborate based cyanylation conjugation chemistry.Refer WO2013/114268.

The 4-PPY formed as a by-product of CPPT cyanylation conjugation chemistry during preparation of polysaccharide- protein conjugates has basic ring of pyrrolidinopyridine which has non-polar characteristics. In a bulk conjugate proteins have relatively less aqueous solubility than polysaccharide molecules. The polysaccharide protein conjugates have high molecular weight (above 900KDa). The molecules of high molecular weight are known to further reduce solubility. This physical attribute coupled with low temperature makes the polysaccharide-protein conjugate molecules in aqueous to orient in micellar form wherein each conjugate bulk is stored at or above 0.3 mg/ ml concentration. Particularly,the location of 4-PPY as residual in micellar polysaccharide-protein conjugate solution is thought to be in the protein core of micelle. Such entrapment of 4PP in micellar form causes great difficulty in releasing 4-PPY at normal temperature when estimating 4-PPY for in-process samples.

Gas chromatography(GC) based method for 4-PPY purity assessment in pure 4-PPY sample is known .Refer WeylChem GmbH, Mannheim fine chemicals/APIs booklet.Use of GC method for quantification of 4-PPY in polysaccharide-protein conjugate vaccine or bulk polysaccharide-protein conjugate has not been reported.However it is known that GC method is not suitable for in-process analysis where immediate estimation of residuals is required for following reasons i)requires complex sample preparation ii)long analysis time , iii) additional time for column stabilization and iv) is imprecise due to possibility of interference of sample matrix and appearance of ghost peaks.

None of the prior art methods are applicable to 4-PPY quantification in polysaccharide-protein conjugate vaccine and polysaccharide protein conjugate bulk.

The present invention arises from the surprising finding that it is possible to quantify 4-PPY in polysaccharide- protein conjugate vaccine and polysaccharide-protein conjugate bulk by utilizing a novel REVERSED Phase - HPLC based assay that comprises of heating sample containing polysaccharide protein conjugate at a temperature between 40 °C and 90°C for a duration between 1 min and 3hrs followed by RP-HPLC.Sample heating step of instant method is postulated to increase the diffusion of 4-PPY into non-micellar regions thereby resulting in collapse of micelles of polysaccharide-protein conjugates.Further said higher temperatures are also expected to increase the solubility of 4-PPY and release ionically associated 4-PPY in aqueous bulk. Heating step was found to provide correct concentration of 4-PPY whereas estimation of 4-PPY without heating was found to provide 4 to 5 times less 4-PPY concentration.

Present invention eliminates the foregoing shortcomings of the prior art methods of assaying for 4-PPY in polysaccharide-protein conjugate vaccine and polysaccharide-protein conjugate bulk by providing a rapid and reproducible (RP-HPLC) based method for quantifying residual 4-pyrrolidinopyridine(4-PPY) in polysaccharide- protein conjugate vaccines prepared by cyanylation conjugation chemistry using 1-Cyano-4- pyrrolidinopyridinium tetrafluorborate (CPPT).

### Summary of Invention:

The invention relates to a novel, rapid and reproducible (RP-HPLC) method for quantifying residual 4-pyrrolidinopyridine(4-PPY) in polysaccharide- protein conjugate vaccines prepared by cyanylation conjugation chemistry using 1-Cyano-4- pyrrolidinopyridinium tetrafluorborate (CPPT).

Instant assay for 4PPY quantification in polysaccharide-protein conjugate samples provides following advantages:
1.Said method is applicable for quantification of 4-PPY in any polysaccharide-protein conjugates(having capsular polysaccharides from Streptococcus Pneumoniae, Neisseria meningitidis , Haemophilus influenzae)prepared by using CPPT conjugation chemistry.
2.It can be utilized for 4PPY quantification for in-process(bulk polysaccharide-protein conjugate) as well as final polysaccharide-protein conjugate vaccines.
3.It has a limit of detection for 4-PPY of about 5ng/ml.
4.It is a rapid method with a total duration of 8 minutes.
5.It is reproducible.
6.It does not require any tedious sample preparation.
7.None of the sample matrix interferes with the 4-PPY peak.

### Description of the figures

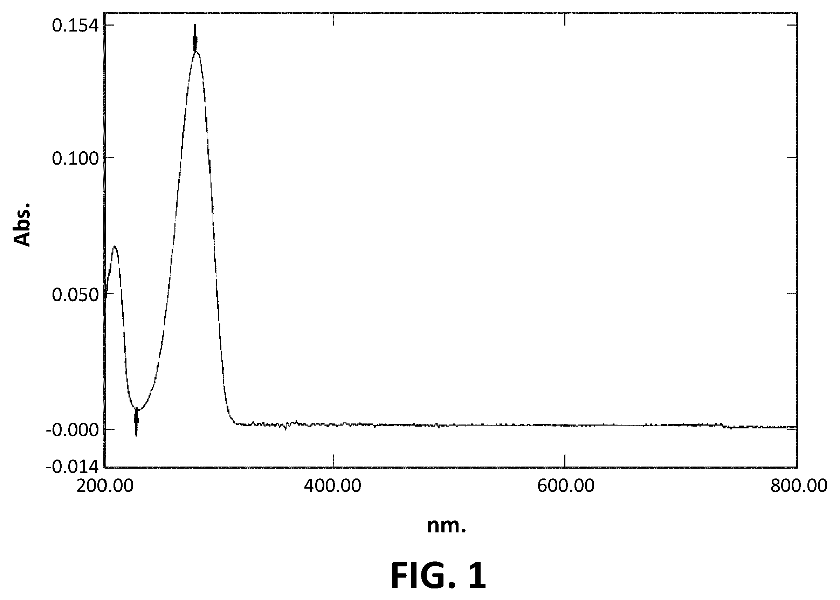

Figure 1: 4-Pyrrolidinopyridine (4-PPY) standard scanned in the UV range using UV-Visible spectrophotometer shows absorption maxima at about 280nm.

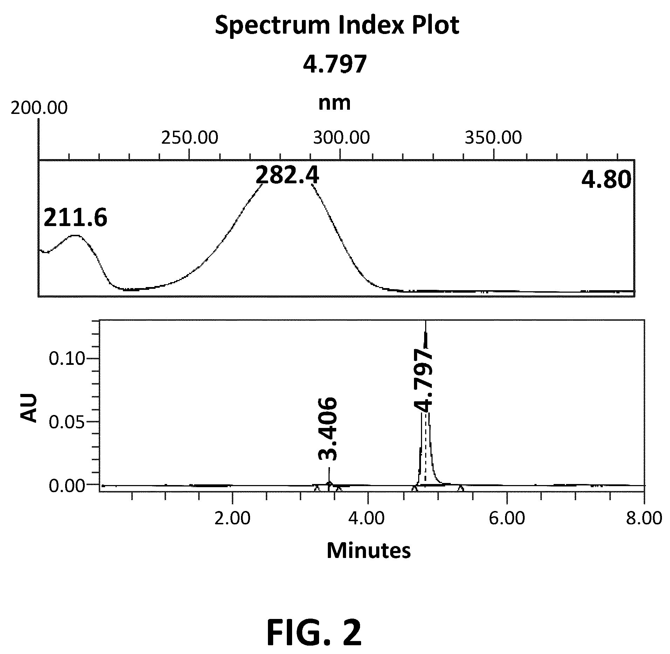

Figure 2: 4-Pyrrolidinopyridine (4-PPY) standard injected into the HPLC with Photodiode array (PDA) detector shows absorption maxima at about 280nm.

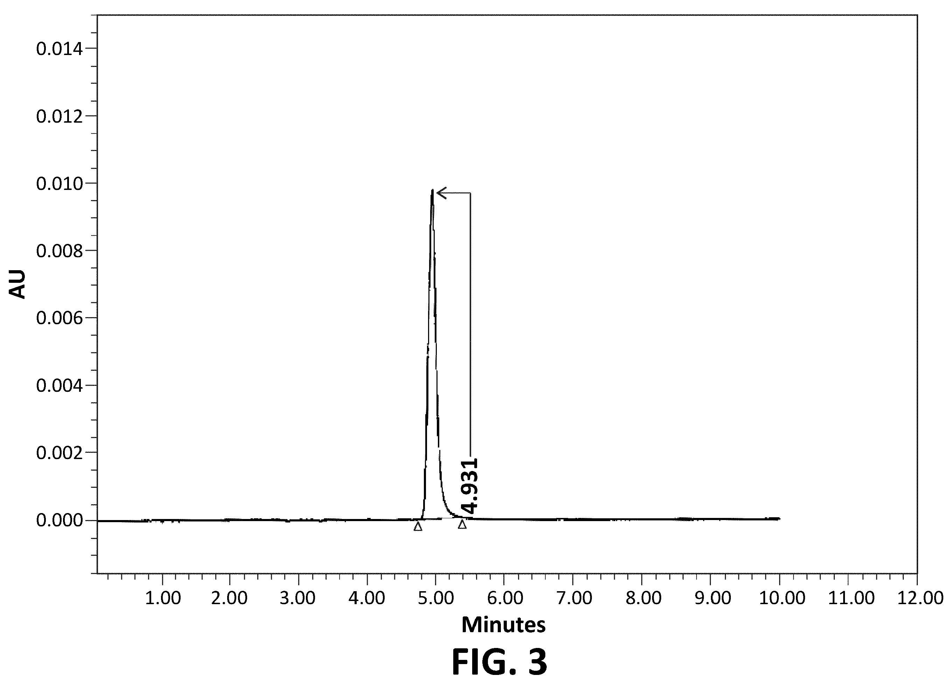

Figure 3: Standard Chromatogram of 4-PPY (1 ppm)

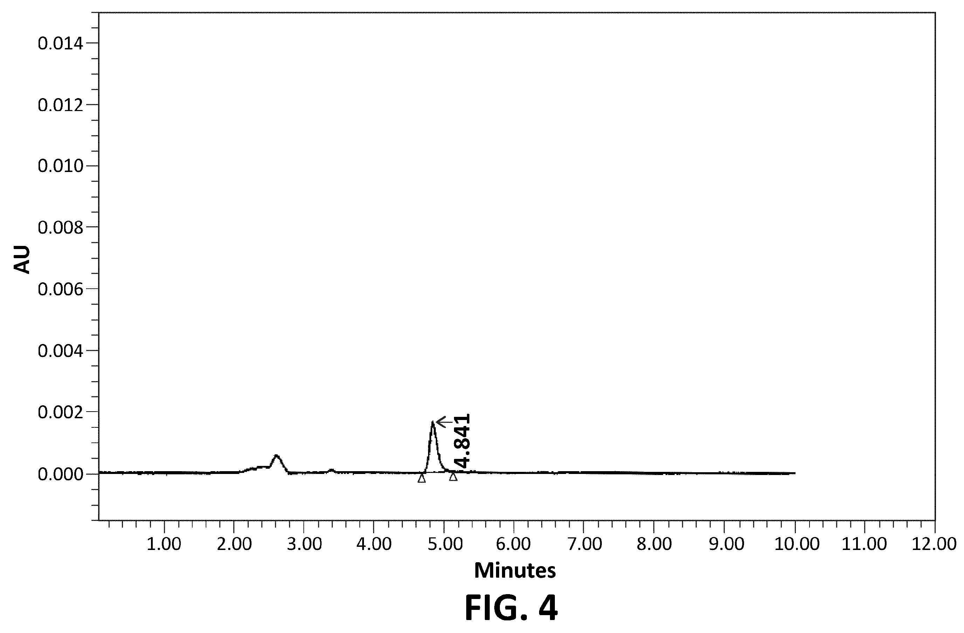

Figure 4: Men W-CRM conjugate injected as such (Initial)

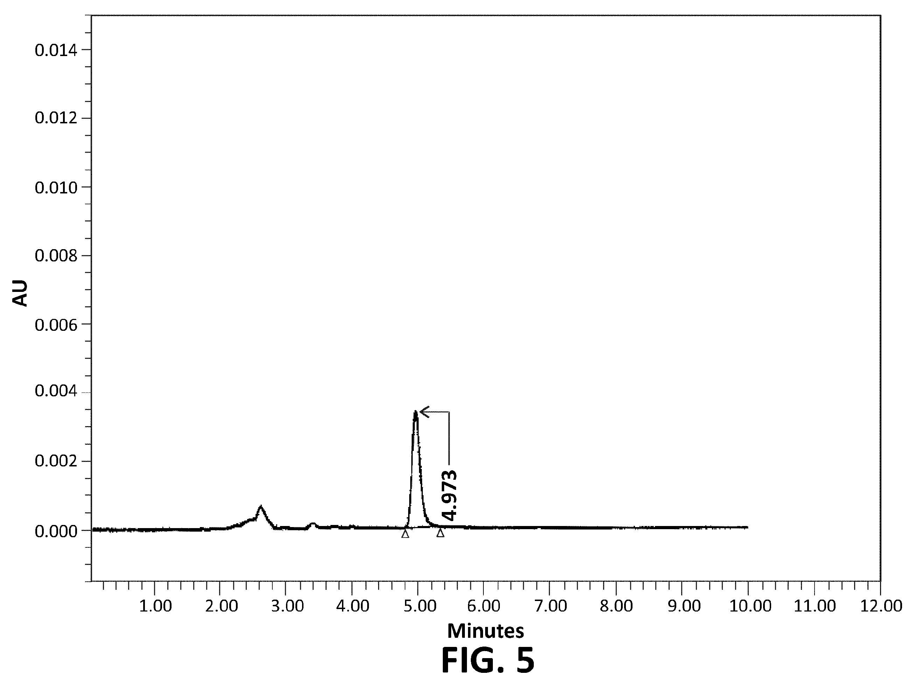

Figure 5: Men W-CRM conjugate heated at 45°C for 3hrs.

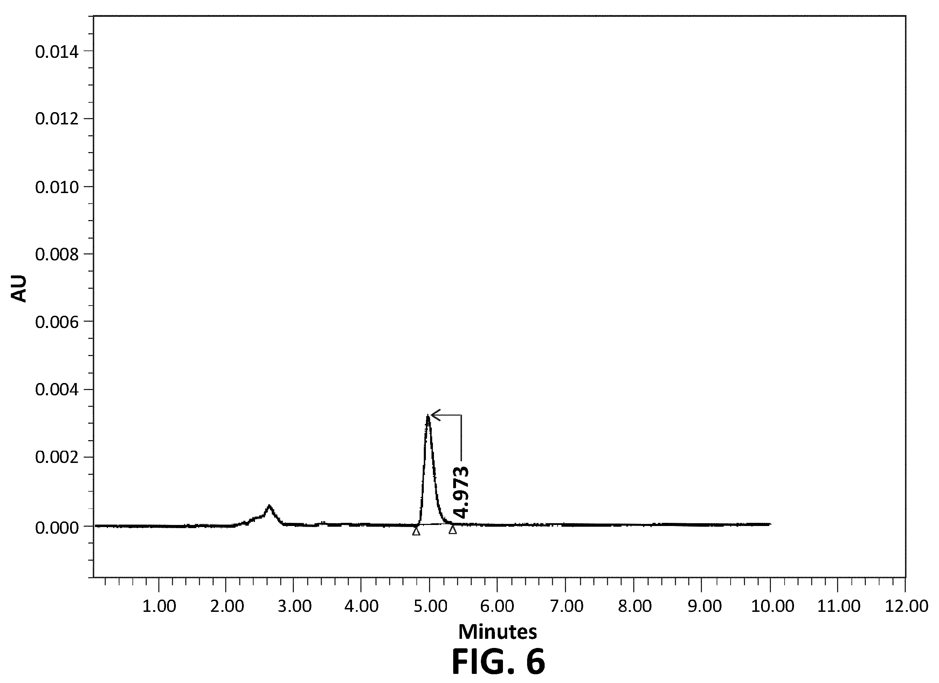

Figure 6 :Men W-CRM conjugate heated at 60°C for 3hrs.

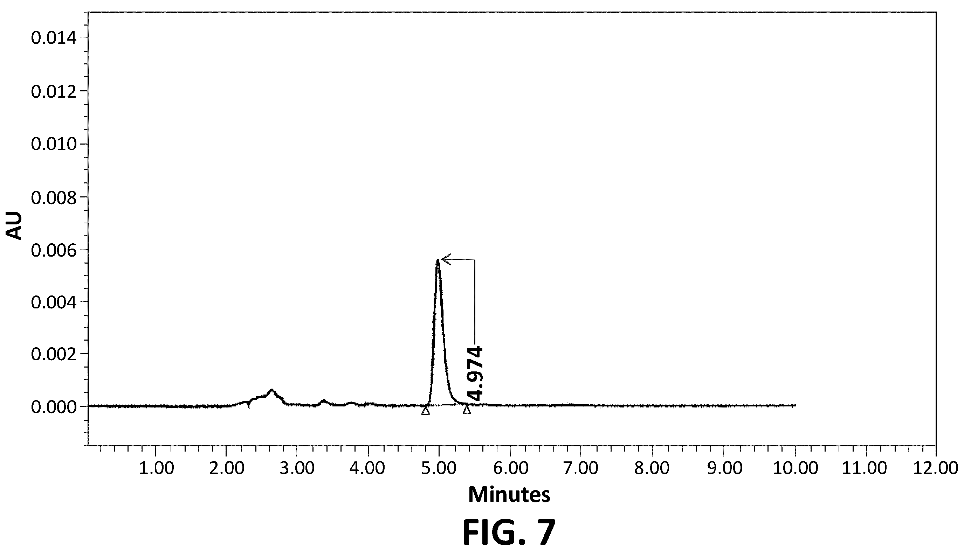

Figure 7 :Men W-CRM conjugate heated at 90°C for 3hrs.

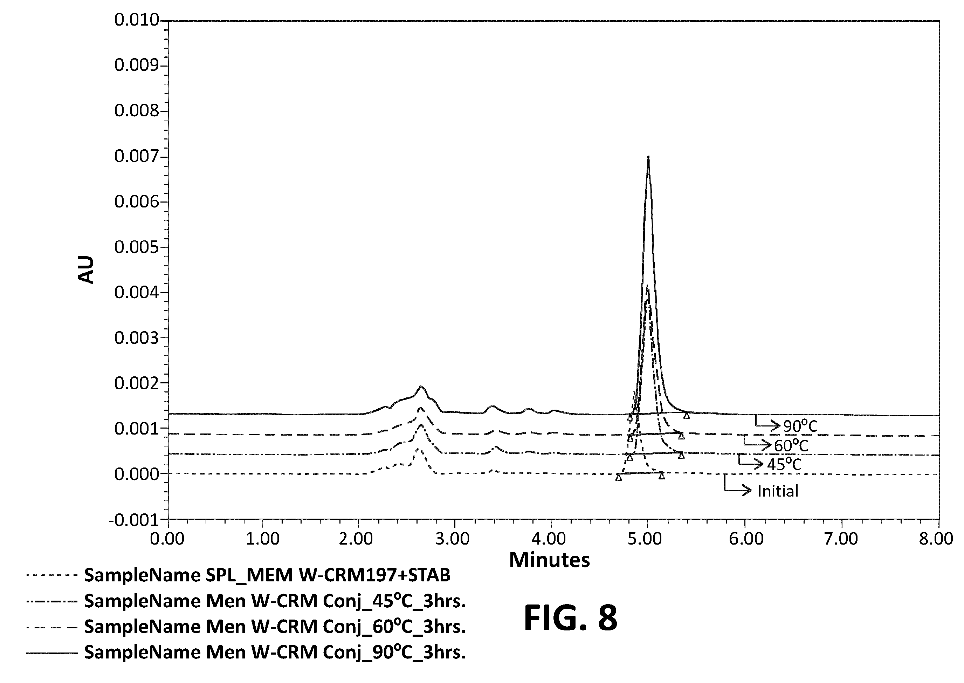

Figure 8 :Overlay chromatograms of Men W-CRM conjugate.

### Detailed Description

An important aspect of the instant invention is that said 4-PPY quantification assay comprises of following steps:
1.Preparation of Trifluoroacetic acid having concentration between 0.01 to 0.1% in water followed by mixing of solution.
2.Preparation of mobile phase containing Trifluoroacetic acid and Acetonitrile in a ratio selected from 80:20,50:50 and 90:10 having a final pH between 2 and 2.4.
3.Saturation of analytical Reversed-Phase High-Performance Liquid Chromatography (RP-HPLC) column.
4.Setting column conditions of temperature at about 25° C,flow rate between 0.8 and 1.2 mL/min,wavelength of 280nm.
5.Injection of known standard solution of 4-PPY(1ppm).
6.Subjecting polysaccharide protein conjugate samples (in-process or final vaccine) to a temperature between 40°C and 90°C for a duration between 1 min and 3hrs
7.Injecting preheated sample of about 10 µL
8.estimation of residual 4-PPY.

Thus, in a preferred embodiment, the invention relates to a RP-HPLC based assay for quantification of 4PPY comprising of a pre-heating step wherein heating is performed at a temperature between about 40°C and about 90°C, more preferably between about 45°C and about 90°C, and even more preferably between about 50°C and about 80°C, most preferably between about 60°C and about 90°C.

Another aspect of instant invention is that said heating of polysaccharide-protein conjugate is carried for a duration between about 1 min and 3hrs, more preferably between about 1 hr and about 2 hr,and even more preferably between about 90 minutes and 3hr, most preferably between 60 minutes and 90 minutes.

An important aspect of the invention is that said superficially porous silica-based reversed-phase support can be derivatized with C4, C8, C18, phenyl or cyano moieties, more preferably reverse phase support can be selected from C-18 USP L1 and C-8 USP L7,most preferably C-8 USP L7.

Another aspect of the instant invention is that said C-8 HPLC column can have dimensions selected from 150x4.6mm,250x4.6mm and 300 x4.6mm.

The mobile phase can consist of an organic modifier and an organic acid wherein the organic modifier can be selected from the group consisting of acetonitrile and methanol whereas the organic acid can be selected from the group consisting of trifluoroacetic acid and heptafluorobutyric acid.

A further embodiment of the instant invention is that mobile phase consists of Trifluoroacetic acid and Acetonitrile in a ratio selected from 80:20,50:50 and 90:10,most preferably 80:20.

Preferably, RP-H PLC uses an analytical column with isocratic mode wherein column temperature can be between 20°C and 25°C and flow rate can be between 0.8 and 1.2 mL/min.

An important embodiment of instant invention is that the 4-PPY content estimated by instant method employing heating step prior to RP-HPLC is about 4 to 15 fold more as compared to the 4-PPY content obtained with direct RP-HPLC.

A second aspect of the instant invention is that said quantification assay for 4-PPY can be utilized for monovalent conjugates or multivalent meningococcal polysaccharide protein conjugate vaccine compositions comprising capsular saccharide from serogroups X and capsular saccharide from A, C, W135 and Y prepared by using 1-Cyano-4-pyrrolidinopyridinium tetrafluorborate based cyanylation conjugation chemistry.

Yet another aspect of the invention is that above method for 4PPY quantification can be applicable to all polysaccharide-protein conjugate vaccines wherein said polysaccharide is a bacterial capsular polysaccharide selected from the group consisting of Neisseria meningitidis groups A ,C , W135 Y and X, Haemophilus influenzae,Group A Streptococcus, Group B Streptococcus, Streptococcus pneumoniae serotypes 1, 2, 3, 4, 5, 6A, 6B,7F,9A,9V,9N,10A, 11A ,12F,14,15B, 17F, 18C, 19A , 19F, 20, 22F,23F ,33F and 45, Escherichia coli, Francisella tularensis, Klebsiella, Moraxella catarrhalis, Porphyromonas gingivalis, Pseudomonas aeruginosa, Burkholderia cepacia, Salmonella typhi , Salmonella typhimurium, Salmonella paratyphi, Shigella dysenteriae, Shigella flexneri, Shigella sonnei, Vibrio cholera,Enterococcus faecalis, Enterococcus faecium, Mycobacterium tuberculosis, Staphylococcus aureus and Staphylococcus epidermidis.

The carrier protein of said polysaccharide-protein conjugates can be selected from a group of but not limited to CRM 197,diphtheria toxoid,tetanus toxoid, fragment C of tetanus toxoid, pertussis toxoid,protein D of H. influenzae, E. coli LT, E: coli ST, and exotoxin A from Pseudomonas aeruginosa, outer membrane complex c (OMPC), porins, transferrin binding proteins, pneumolysin, pneumococcal surface protein A (PspA), pneumococcal adhesin protein (PsaA),pneumococcal PhtD, pneumococcal surface proteins BVH-3 and BVH-11 , protective antigen (PA) of Bacillus anthracis and detoxified edema factor (EF) and lethal factor (LF) of Bacillus anthracis, ovalbumin, keyhole limpet hemocyanin (KLH), human serum albumin, bovine serum albumin (BSA) and purified protein derivative of tuberculin (PPD),particularly CRM197.

The following examples are intended to illustrate one embodiment now known for practicing the invention, but the invention is not to be considered limited to these examples

### Examples

### I) Preparation of MenA-TT Conjugate

### Polysaccharide preparation:

MenA-TT conjugate was prepared by cyanylation and carbodiimide chemistry. The native polysaccharide was sized reduced by mechanical means. The sized reduced PS (100-120kD on SEC-HPLC) was diafiltered & concentrated on 10kD cuttoff membrane. To 6mg/ml PS, freshly prepared solution of CPPT(dissolved 114 mg/ml in acetonitrile) added the pH was shifted to 9.5 with NaOH solution and hold for upto 5 min. Adipic acid dihydrazide (ADH) dissolved 100mg/ml in water was added to the PS solution. The reaction was stirred for 1 hrs at 25°C. The reaction was quenched by addition of excess glycine solution. The reaction mixture was diafiltered on 10kD TFF in MES buffer to remove residuals and unreacted components. The sample was stored at 2-8°C until use. The PS content was measured by phosphorous assay and extent of derivatization was measured by TNBS assay.

### Carrier Protein (TT) Preparation:

Tetanus toxoid was chromatographically purified to remove aggregates that might cause adverse effect in the conjugation process. Total TT load was 5-10% v/v of packed column (toyopearl HW-65F, Tosoh biosciences) with 30cm/hr linear flow rate. The fractions were collected on the basis of chromatogram obtained on UV detector of the chromatography system. The fractions were pooled on the basis of aggregate removal seen on SEC-HPLC. The collected fractions were subjected to concentration-diafiltration on 30kD TFF in MES buffer. The protein content was measured by Lowry assay.

### Conjugation of Men A to TT:

The ADH-derivatized PS, purified TT and EDAC (1-ethyl-3-(3-dimethyl-aminopropyl) carbodiimide) were mixed the ratio of 1:0.8:1.5 by weight under stirring condition. The reaction was monitored on HPLC for protein conversion. The reaction was quenched after 3 hours by raising pH with 10mM PB+EDTA pH 8.0 buffer.

### Conjugate Purification:

The conjugate was purified on 300kD cut off membrane by passing 30 volumes of 100mM PBS and followed by 30 volume10mM Tris pH 7.2. The sucrose-citrate stabilizer was added to bulk conjugate and stored below -20°C until use. The purified bulk conjugate was analyzed for total and unbound (free) PS, Total and unbound(free) protein and residual 4-PPY in the conjugate bulk.

**Table 1**

| **Test Sample** | **Total PS mg/ml** | **Total Protein mg/ml** | **% Free PS** | **% Free Protein** |
|---|---|---|---|---|
| Men A-TT Purified Conjugate | 1.1 | 2.23 | 4.10 | 2.27 |

### II) Preparation of MenC-CRM197 Conjugate

### Polysaccharide preparation:

MenC PS was size-reduced by high pressure homogenization to 75-100kDa on SEC -HPLC, which is 40-50kD on MALLS and the PS was then diafiltered and concentrated on 10kD cuttoff membrane

### Carrier Protein (CRM197) Preparation:

In order to conjugate Men C to CRM197, the carrier protein was derivatized by ADH linker prior to conjugation.
The native CRM197 was allowed to react with ADH linker in presence of EDAC (1-ethyl-3-(3-dimethyl-aminopropyl) carbodiimide) in MES buffer. The reaction was then quenched by increasing pH by sodium carbonate solution under stirring for 15min. The ADH-derivatized CRM197 was then diafiltered on 10kD cuttoff membrane using 10mM carbonate buffer pH 9.5 by passing about 35 volumes of buffer. The sample was submitted for content analysis and extent of derivatization.

### Conjugation of Men C to CRM197:

MenC PS was mixed with CPPT( in acetonitrile) and then with ADH-derivatized CRM197 in the ratio of 1:0.7:1.25 by weight under stirring condition. The reaction was monitored on HPLC. The reaction was quenched after 4 hours by addition of excess glycine solution.

### Conjugate Purification:

The crude conjugate was purified on 300kD cut off membrane by passing 30 volumes of 100mM PBS and followed by 10 volume 10mM Tris pH 7.2 and then 20 volumes of 10mM Tris on 100kD cut off membrane. The sucrose-citrate stabilizer mixture was added to the bulk conjugate and stored below -20°C until use.
The purified bulk conjugate was analyzed for total and unbound (free) PS, Total and unbound(free) protein and residual 4-PPY in the conjugate bulk.

**Table 2**

| **Test Sample** | **Total PS mg/ml** | **Total Protein mg/ml** | **% Free PS** | **% Free Protein** |
|---|---|---|---|---|
| Men C-CRM197 Purified Conjugate | 1.10 | 2.11 | <2.16 | 4.5 |

### III) Preparation of MenY-CRM197 Conjugate

### Polysaccharide preparation:

Men Y PS sizing was carried out by chemical means. The chemically sized PS was targeted to achieve size of 75-100kDa on SEC-HPLC and then the polysaccharide was diafiltered on 10kD cuttoff membrane.

### Carrier Protein (CRM197) Preparation:

The carrier Protein derivatization process was same as described above for MenC-CRM conjugate.

### Conjugation of Men Y to CRM197:

MenY PS was mixed with CPPT (113 mg/ml in acetonitrile), pH adjusted to 9.5 immediately after addition and then with ADH-derivatized CRM197 in the ratio of 1:1.5:1.25 by weight under stirring condition. The reaction was monitored on HPLC. The reaction was quenched by addition of excess glycine solution.

### Conjugate Purification:

The conjugate was purified on 300kD cut off membrane by passing 30 volumes of 100mM PBS and followed by 10 volume10mM Tris pH 7.2 and then 20 volumes of 10mM Tris on 100kD cut off membrane. The sucrose-citrate stabilizer mixture was added to the bulk conjugate and stored below -20°C until use.
The purified bulk conjugate was analyzed for total and unbound (free) PS, Total and unbound(free) protein and residual 4-PPY in the conjugate bulk.

**Table 3**

| **Test Sample** | **Total PS mg/ml** | **Total Protein mg/ml** | **% Free PS** | **% Free Protein** |
|---|---|---|---|---|
| Men Y-CRM197 Purified Conjugate | 1.46 | 3.59 | <4.43 | <4.09 |

### IV) Preparation of MenW-CRM197 Conjugate

### Polysaccharide preparation:

In case of Men W PS, sizing was carried out by mechanical means and sizing continued until the PS size was reached to 120-130 kD on SEC-HPLC and then the polysaccharide was diafiltered-concentrated on 10kD cuttoff membrane.

### Carrier Protein (CRM197) Preparation:

The carrier Protein derivatization process was same as described above for MenY-CRM197 conjugate.

### Conjugation of Men W to CRM197:

MenW PS was mixed with CPPT (113 mg/ml in acetonitrile), pH adjusted to 9.5 immediately after addition and then ADH-derivatized CRM197 was added in the ratio of 1:0.8:0.8 by weight under stirring condition. The reaction was monitored on HPLC for protein peak to disappear. The reaction was quenched after 4 hours by addition of glycine buffer.

### Conjugate Purification:

The conjugate was purified on 300kD cut off membrane by passing 30 volumes of 100mM PBS and followed by 10 volume10mM Tris pH 7.2 and then 20volumes of 10mM Tris on 100kD cut off membrane. The sucrose-citrate stabilizer mixture added to the bulk conjugate and stored below -20°C until use.The purified bulk conjugate was analyzed for total and unbound (free) PS, Total and unbound(free) protein and residual 4-PPY in the conjugate bulk.

**Table 4**

| **Test Sample** | **Total PS mg/ml** | **Total Protein mg/ml** | **% Free PS** | **% Free Protein** |
|---|---|---|---|---|
| Men W-CRM197 Purified Conjugate | 1.53 | 2.54 | <4.25 | <4.08 |

### V) Preparation of MenX-TT Conjugate

### Polysaccharide preparation:

MenX-TT conjugate was prepared by cyanylation and carbodiimide chemistry. The native polysaccharide was size- reduced by mechanical means. The size-reduced PS (120-150kD on SEC-HPLC) was diafiltered & concentrated on 10kD cuttoff membrane. To 20 mg/ml PS, freshly prepared solution of CPPT (also known as CPPT dissolved 114 mg/ml in acetonitrile) was added and the pH was shifted to 9.5 with NaOH solution and incubated for 2-4 min. Adipic acid dihydrazide (ADH) was dissolved 100mg/ml in water and added to the PS solution. The reaction was stirred for 3 hrs at 25°C. The reaction was quenched by addition of excess glycine solution. The reaction mixture was diafilterted in MES buffer to remove residuals and unreacted components. The sample was stored at 2-8°C until use. The PS content was measured by phosphorous assay and extent of derivatization was measured by TNBS assay.

### Carrier Protein (TT) Preparation:

The carrier Protein preparation process was similar as described above.

### Conjugation of Men X to TT:

The ADH-derivatized PS, purified TT and EDAC (1-ethyl-3-(3-dimethyl-aminopropyl) carbodiimide) were mixed in a ratio of 1:1.5:0.7 by weight under stirring condition. The reaction progress was monitored on HPLC. The reaction was quenched after 3hrs by raising pH with 10mM PB+EDTA pH 8.0 buffer.

### Conjugate Purification:

The conjugate was purified on 300kD cut off membrane by passing 30 volumes of 100mM PBS and followed by 30 volume10mM Tris pH 7.2. The purified bulk conjugate was analyzed for total and unbound (free) PS, total and protein and residual 4-PPY in the conjugate bulk.

**Table 5**

| **Test Sample** | **Total PS mg/ml** | **Total Protein mg/ml** | **% Free PS** |
|---|---|---|---|
| Men X-TT Purified Conjugate | 1.08 | 2.44 | 7.87 |

### VI) Preparation of Pneumococcal 23F-CRM197 Conjugate

### Polysaccharide preparation:

In case of Pneumococcal 23F Ps, sizing was carried out by mechanical means and sizing was continued until the PS size reached to 150-170 kD on SEC-HPLC and then the polysaccharide was diafiltered-concentrated on 10kD cuttoff membrane.

### Carrier Protein-CRM197 Preparation:

CRM197 protein content 31mg/ml was used for conjugation.

### Conjugation of Pneumococcal 23F-CRM197

Pneumococcal 23F PS was subjected mixed with CPPT( in acetonitrile) and then native CRM197 in the ratio of 1:1.25:1.5 by weight under stirring condition. The reaction was monitored on HPLC. The reaction was quenched after 2 hrs by addition of excess glycine solution.

### Conjugate Purification:

The conjugate was purified on 300kD cut off membrane by passing 30 volumes of 10mM PBS and followed by 20 volume10mM Tris pH 7.2. The purified bulk conjugate was analyzed for total and unbound (free) PS, total and unbound(free) protein and residual 4-PPY in the conjugate bulk.

**Table 6**

| **Test Sample** | **Total PS mg/ml** | **Total Protein mg/ml** | **% Free PS** | **% Free Protein** |
|---|---|---|---|---|
| Pneumococcal 23F-CRM₁₉₇ Conjugate | 0.94 | 1.91 | 7.43 | 1.79 |

### VII) Preparation of Pneumococcal conjugate 23F-CRM197-ADH Conjugate

### Polysaccharide preparation:

In case of Pneumococcal 23F Ps, sizing was carried out by mechanical means and sizing was continued until the PS size was reached to 150-170 kD on SEC-HPLC and then the polysaccharide was diafiltered-concentrated on 10kD cuttoff membrane.

### Carrier Protein (CRM197) Preparation:

In order to conjugate Pneumococcal 23F Ps to CRM197, the carrier protein was derivatized by ADH linker prior to conjugation.
The native CRM197 was allowed to react with ADH linker in presence of EDAC (1-ethyl-3-(3-dimethyl-aminopropyl) carbodiimide) in MES buffer. The reaction was then quenched by increasing pH by sodium carbonate solution under stirring for 15min. The ADH-derivatized CRM197 was then diafiltered on 10kD cuttoff membrane using 10mM carbonate buffer pH 9.5 by passing about 35 volumes of buffer. The sample was submitted to content analysis and extent of derivatization.

### Conjugation of Pneumococcal 23F-CRM197:

Pneumococcal 23F PS was mixed with CPPT( in acetonitrile) and then ADH-derivatized CRM197 in the ratio of 1:1.25:1.25 by weight under stirring condition. The reaction was monitored on HPLC. The reaction was quenched after 3 hours by addition of excess glycine solution.

### Conjugate Purification:

The crude conjugate was purified on 300kD cut off membrane by passing 30 volumes of 10mM PBS and followed by 20 volume10mM Tris pH 7.2. The purified bulk conjugate was analyzed for total and unbound (free) PS, total and unbound(free) protein and residual 4-PPY in the conjugate bulk.

**Table 7**

| **Test Sample** | **Total PS mg/ml** | **Total Protein mg/ml** | **% Free PS** | **% Free Protein** |
|---|---|---|---|---|
| Pneumococcal 23F-CRM197 Conjugate | 0.87 | 1.58 | 1.67 | 2.11 |

### VIII) Preparation of Pneumococcal 9V-TT Conjugate

### Polysaccharide preparation:

Pneumococcal 9V PS, sizing was carried out by mechanical means and sizing continued until the PS size was reached to 150-170 kD on SEC-HPLC and then the polysaccharide was diafiltered-concentrated on 10kD cuttoff membrane.

### Carrier Protein (TT) Preparation:

Tetanus toxoid was chromatographically purified to remove aggregates that might cause adverse effect in the conjugation process. Total TT load was 5-10% v/v of packed column (toyopearl HW-65F, Tosoh biosciences) with 30cm/hr linear flow rate. The fractions were collected on the basis of chromatogram obtained on UV detector of the chromatography system. The fractions were pooled on the basis of aggregate removal seen on SEC-HPLC. The collected fractions subjected to concentration-diafiltration on 30kD TFF in MES buffer. The protein content was measured **by** Lowry assay.

### Conjugation of Pneumococcal 9V to TT:

Pneumococcal 9V PS was mixed with CPPT(in acetonitrile) and then TT in the ratio of 1:0.7:1.5 by weight under stirring condition at pH 9-10. The reaction progress was monitored on HPLC. The reaction was quenched after 1 hr 30 min by addition of excess glycine solution.

### Conjugate Purification:

The crude conjugate was purified on 300kD cut off membrane by passing 30 volumes of 10mM PBS and followed by 20 volume 10mM Tris pH 7.2. The purified bulk conjugate was analyzed for total and unbound (free) PS, Total and unbound(free) protein and residual 4-PPY in the conjugate bulk.

**Table 8**

| **Test Sample** | **Total PS mg/ml** | **Total Protein mg/ml** | **% Free PS** |
|---|---|---|---|
| Pneumococcal 9V-TT Conjugate | 0.80 | 1.44 | <2 |

### IX) Preparation of Pneumococcal 9V-CRM197 Conjugate

### Polysaccharide preparation:

Pneumococcal 9V PS, sizing was carried out by mechanical means and sizing continued until the PS size was reached to 150-170 kD on SEC-HPLC and then the polysaccharide was diafiltered-concentrated on 10kD cuttoff membrane.

### Carrier Protein (CRM197) Preparation:

CRM197 protein content 31mg/ml was used for conjugation.

### Conjugation of Pneumococcal 9V to CRM197:

Pneumococcal 9V PS was mixed with CPPT(in acetonitrile) and then CRM197 in the ratio of 1:1:1.5 by weight under stirring condition. The reaction was monitored on HPLC. The reaction was quenched after 2 hours by addition of excess glycine solution.

### Conjugate Purification:

The conjugate was purified on 300kD cut off membrane by passing 30 volumes of 10mM PBS and followed by 10 volume10mM Tris pH 7.2 The purified bulk conjugate was analyzed for total and unbound (free) PS, total and unbound (free) protein and residual 4-PPY in the conjugate bulk.

**Table 9**

| **Test Sample** | **Total PS mg/ml** | **Total Protein mg/ml** | **% Free PS** | **% Free Protein** |
|---|---|---|---|---|
| Pneumococcal 9V-CRM197 Conjugate | 1.01 | 1.98 | <2 | 2.64 |

### X) Preparation of Pneumococcal 14-CRM197-ADH Conjugate

### Polysaccharide preparation:

In case of Pneumococcal 14 Ps, sizing was carried out by mechanical means and sizing was continued until the PS size reached to 100-120 kD on SEC-HPLC and then the polysaccharide was diafiltered-concentrated on 10kD cuttoff membrane.

### Carrier Protein (CRM197) Preparation:

In order to conjugate Pneumococcal 14 Ps to CRM197, the carrier protein was derivatized by ADH linker prior to conjugation.

The native CRM197 was allowed to react with ADH linker in presence of EDAC (1-ethyl-3-(3-dimethyl-aminopropyl) carbodiimide) in MES buffer. The reaction was then quenched by increasing pH by sodium carbonate solution under stirring for 15min. The ADH-derivatized CRM197 was then diafiltered on 10kD cuttoff membrane using 10mM carbonate buffer pH 9.5 by passing about 35 volumes of buffer. The sample was submitted to content analysis and extent of derivatization.

### Conjugation of Pneumococcal 14-CRM₁₉₇:

Pneumococcal 14 PS was mixed with CPPT( in acetonitrile) and then ADH-derivatized CRM197 in the ratio of 1:1.25:1.25 by weight under stirring condition. The reaction progress was monitored on HPLC. The reaction was quenched after 3 hours by addition of excess glycine solution.

### Conjugate Purification:

The crude conjugate was purified on 300kD cut off membrane by passing 30 volumes of 10mM PBS and followed by 20 volume10mM Tris pH 7.2. The purified bulk conjugate was analyzed for total and unbound (free) PS, total and unbound(free) protein and residual 4-PPY in the conjugate bulk.

**Table 10**

| **Test Sample** | **Total PS mg/ml** | **Total Protein mg/ml** | **% Free PS** | **% Free Protein** |
|---|---|---|---|---|
| Pneumococcal 14-CRM197 Conjugate | 1.22 | 1.43 | 5.29 | <1 |

### Analysis of Meningococcal conjugate for 4-PPY content:

Meningococcal conjugate(s) (A C W Y X) were analysed for the 4-PPY content. 4-PPY quantification was carried out as follows:
1.Trifluoroacetic acid (0.05%) was prepared in water followed by mixing of solution.
2.Mobile phase containing Trifluoroacetic acid and Acetonitrile in a ratio 80:20 having a final pH between 2 and 2.4 was prepared.
3.Analytical Reversed-Phase High-Performance Liquid Chromatography (RP-HPLC) column was saturated.
4.Column temperature was set at about 25° C,flow rate was between 0.8 and 1.2 mL/min.
5.Known standard solution of 4-PPY(1 ppm)was injected.
6.Polysaccharide protein conjugate samples (in-process or final vaccine)were subjected to 90°C for a duration between of 3hrs.
7.preheated sample 10 µL was injected.
8.4-PPY was estimated at a wavelength of 280nm.

**Table 11- 4-PPY content in Meningococcal conjugate(s) (A C W Y X)**

| **Samples** | **4-PP (ng/mL)** | | | |
|---|---|---|---|---|
| | Initial | Samples heated at 45°C for 3hrs. | Samples heated at 60°C for 3hrs. | Samples heated at 90°C for 3hrs. |
| Men A-TT Conjugate | 8 | 19 | 23 | 27 |
| MEN C-CRM Conjugate | 126 | 352 | 384 | 654 |
| MEN Y-CRM Conjugate | 523 | 1067 | 1135 | 1994 |
| MEN W-CRM Conjugate | 175 | 396 | 422 | 694 |
| MEN X-TT Conjugate | 6 | 29 | 31 | 51 |

It was observed that when the sample was exposed to elevated temperatures i.e. 45°C, 60°C, 90°C for about 3hrs 4-PP content ted was found to be 3 to 4 times more at 90°C as compared to the 4-PPY content obtained without heating.

### Analysis of Pneumococcal conjugate(s) for 4-PPY content:

Pneumococcal conjugate(s) (9V, 14,23) were analysed for the 4-PP content.

**Table 12- 4-PPY content in Pneumococcal conjugate(s)(9V,14,23F)**

| **Samples** | **4**-**PPY (ng/mL)** | | | |
|---|---|---|---|---|
| | Initial | Samples heated at 45°C for 3hrs. | Samples heated at 60°C for 3hrs. | Samples heated at 90°C for 3hrs. |
| PN9V-CRM Conjugate | 139 | 274 | 587 | 2458 |
| PN9V-TT Conjugate | 188 | 429 | 848 | 4425 |
| PN14-CRM Conjugate | 113 | 206 | 446 | 1585 |
| PN23F-CRM Conjugate-1 | 512 | 545 | 1435 | 3608 |
| PN23F-CRM Conjugate-2 | 378 | 669 | 1075 | 2664 |

It was observed that when sample(s) were exposed to elevated temperature i.e. 45°C, 60°C, 90°C for about 3hrs the estimated 4-PPY content was found to be 10 to 15 times more at 90°C as compared to that obtained for sample(s) without heating.

It is to be understood that typical methods of RP-HPLC technology have been applied, and that a person skilled in the art of RP-HPLC and HPLC is well aware of minor adjustments that would not alter the results to be obtained, such as different measurements at other suitable wavelengths or by the use of other column material that would not severely alter the results obtained by the invention.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims.

## Claims

1. An improved method for assaying a sample suspected to contain 4-pyrrolidinopyridine(4-PPY)comprising the steps of:
(i)incubating sample containing 4-PPY at a temperature between 40° C and 90° C for a duration between 1 hr and 3hrs.
(ii) subjecting pre-heated sample to a reversed phase high performance liquid chromatography.
(iii) detecting any 4-pyrrolidinopyridine(4-PPY).

2. A method according to claim 1, wherein the sample is a polysaccharide-protein conjugate prepared by cyanylation conjugation chemistry using 1-Cyano-4- pyrrolidinopyridinium tetrafluorborate (CPPT).

3. A method according to claim 1, wherein the heating in step (i) is performed at a temperature between 60° C and 90° C for a duration between 60 minutes and 180 minutes.

4. A method according to claim 3, wherein the heating in step (i) is performed at 90° C for a duration of 180 minutes.

5. A method according to claim 1, wherein the reversed phase HPLC uses an Isocratic mode.

6. A method according to claim 1 , wherein the reversed-phase analytical column is a superficially porous silica-based and the column support is selected from a group consisting of C4, C8, C18, phenyl and cyano.

7. A method according to claim 6 ,wherein the analytical column is C8 (as USP L7).

8. A method of claim 1,wherein mobile phase for reversed phase HPLC consists of an organic modifier and an organic acid.

9. A method of claim 8, wherein the organic modifier is selected from the group consisting of acetonitrile and methanol.

10. A method of claim 8, wherein the organic acid is selected from the group consisting of trifluoroacetic acid and heptafluorobutyric acid.

11. A method of claim 8 , wherein mobile phase consists of trifluoroacetic acid and acetonitrile in a ratio selected from 80:20,50:50 and 90:10.

12. A method of claim 11, wherein mobile phase consists of Trifluoroacetic acid and Acetonitrile in a ratio of 80:20.

13. A method according to claim 12 , wherein mobile phase comprises between 0.01 and 0.1% by volume of trifluoroacetic acid.

14. A method according to claim 13, wherein mobile phase comprises 0.05% by volume of trifluoroacetic acid.

15. A method according to claim 1 , wherein the mobile phase of reversed phase-HPLC has a pH between 2 and 2.5.

16. A method according to claim 1 ,wherein the reversed phase-HPLC column temperature is between 20°C and 25°C.

17. A method according to claim 16 ,wherein the column temperature is 25°C.

18. A method of claim 1, wherein the flow rate for reversed phase-HPLC is between 0.8 and 1.2 mL/min.

19. A method of claim 1,wherein the detection is carried at a wavelength of 280nm.

20. A method according to claim 1, wherein the 4-PPY content estimated by subjecting sample to heating prior to RP-HPLC is at least 4 fold more as compared to the 4-PPY content obtained with RP-HPLC without pre-heating the sample.

21. The method of any claim 2-20 wherein the capsular polysaccharide is a polysaccharide selected from a Meningococcal polysaccharide, a Pneumococcal polysaccharide, a Haemophilus influenzae type B polysaccharide, a Vi polysaccharide of Salmonella typhi, or a group B Streptococcus polysaccharide.

22. The method of any claim 2-20 wherein the polysaccharide can be selected from Meningococcal group A polysaccharide, a Meningococcal group C polysaccharide, a Meningococcal group W-135 polysaccharide, a Meningococcal group Y and a Meningococcal group X.

23. The method of any claim 2-20 wherein the polysaccharide can be selected from Pneumococcal group 1, 2, 3, 4, 5, 6A, 6B,7F,8, 9A,9V,9N,10A, 11A,12F,14,15B, 17F, 18C, 19A, 19F, 20, 22F,23F ,33F and 45.

24. The method of any claim 2-23, wherein the carrier protein of the polysaccharide-protein conjugates is a protein selected from the group consisting of tetanus toxoid (TT), fragment C of TT, diphtheria toxoid, CRM197, Pneumolysin, protein D, PhtD, PhtDE,OMPC and N19.

25. A method of any preceding claim, wherein the sample being assayed for 4-PPY comprises of at least one conjugate selected from (a)a conjugate of (i) the capsular saccharide of serogroup A N meningitidis and (ii)tetanus toxoid; (b) a conjugate of (i) capsular saccharide of serogroup C N meningitidis and (ii)CRM197; (c) a conjugate of (i) capsular saccharide of serogroup Y N. meningitidis and (ii) CRM197;(d) a conjugate of (i) capsular saccharide of serogroup W135 N.meningitidis and (ii)CRM197; and (d) a conjugate of (i) capsular saccharide of serogroup X N meningitidis and (ii)tetanus toxoid.

## Patentansprüche

1. Ein verbessertes Verfahren zur Analyse einer Probe, die vermutlich 4-Pyrrolidinopyridin (4-PPY) enthält, das folgende Schritte umfasst:
(i) Bebrütung der 4-PPY-haltigen Probe bei einer Temperatur zwischen 40° C und 90° C für eine Dauer zwischen 1 Stunde und 3 Stunden.
(ii) die vorgewärmte Probe einer Umkehrphasen-Hochleistungs-Flüssigkeitschromatographie unterziehen.
(iii) Erkennen von möglicherweise vorhandenem 4-Pyrrolidinopyridin (4-PPY).

2. Verfahren nach Anspruch 1, wobei die Probe ein Polysaccharid-Protein-Konjugat ist, das durch Cyanylierungs-Konjugations-Chemie unter Verwendung von 1-Cyano-4-pyrrolidinopyridin-Tetrafluorborat (CPPT) hergestellt wird.

3. Verfahren nach Anspruch 1, wobei die Erwärmung in Schritt (i) bei einer Temperatur zwischen 60° C und 90° C für eine Dauer zwischen 60 Minuten und 180 Minuten erfolgt.

4. Verfahren nach Anspruch 3, wobei die Erwärmung in Schritt (i) bei 90° C für eine Dauer von 180 Minuten erfolgt.

5. Verfahren nach Anspruch 1, wobei die Umkehrphasen-HPLC einen isokratischen Modus verwendet.

6. Verfahren nach Anspruch 1, wobei die Umkehrphasen-Trennsäule eine poröse Oberfläche auf Siliziumdioxidbasis aufweist und die stationäre Phase aus einer Gruppe von C4, C8, C18, Phenyl und Cyano ausgewählt wurde.

7. Verfahren nach Anspruch 6, wobei die Trennsäule aus C8 besteht (als USP L7).

8. Verfahren nach Anspruch 1, wobei die mobile Phase für die Umkehrphasen-HPLC aus einem organischen Modifikator und einer organischen Säure besteht.

9. Verfahren nach Anspruch 8, wobei der organische Modifikator aus der Gruppe mit Acetonitril und Methanol ausgewählt wurde.

10. Verfahren nach Anspruch 8, wobei die organische Säure aus der Gruppe mit Trifluoressigsäure und Heptafluorbuttersäure ausgewählt wurde.

11. Verfahren nach Anspruch 8, wobei die mobile Phase aus Trifluoressigsäure und Acetonitril in einem Verhältnis von 80:20, 50:50 und 90:10 besteht.

12. Verfahren nach Anspruch 11, wobei die mobile Phase aus Trifluoressigsäure und Acetonitril in einem Verhältnis von 80:20 besteht.

13. Verfahren nach Anspruch 12, wobei die mobile Phase aus 0,01 bis 0,1 Vol.-% Trifluoressigsäure besteht.

14. Verfahren nach Anspruch 13, wobei die mobile Phase aus 0,05 Vol-% Trifluoressigsäure besteht.

15. Verfahren nach Anspruch 1, wobei die mobile Phase der Umkehrphasen-HPLC einen pH-Wert zwischen 2 und 2,5 hat.

16. Verfahren nach Anspruch 1, wobei die Temperatur der Umkehrphasen-HPLC-Säule zwischen 20°C und 25°C liegt.

17. Verfahren nach Anspruch 16, wobei die Säulentemperatur 25°C beträgt.

18. Verfahren nach Anspruch 1, wobei die Flussrate für die Umkehrphasen-HPLC zwischen 0,8 und 1,2 ml/min. liegt.

19. Verfahren nach Anspruch 1, wobei die Erkennung bei einer Wellenlänge von 280nm durchgeführt wird.

20. Verfahren nach Anspruch 1, wobei der durch Erhitzen der Probe vor der RP-HPLC geschätzte 4-PPY-Gehalt mindestens 4mal so hoch ist im Vergleich zum 4-PPY-Gehalt, der durch RP-HPLC ohne Vorheizen der Probe erhalten wird.

21. Verfahren nach einem der Ansprüche 2-20, wobei das Kapsel-Polysaccharid ein Polysaccharid ist, das aus einem Meningokokken-Polysaccharid, einem Pneumokokken-Polysaccharid, einem Polysaccharid von Haemophilus influenzae-Typ-B, einem Vi-Polysaccharid von Salmonella typhi oder einem Streptokokken-Polysaccharid der Gruppe B ausgewählt wurde.

22. Verfahren nach einem der Ansprüche 2-20, wobei das Polysaccharid aus einem Meningokokken-Polysaccharid der Gruppe A, einem Meningokokken-Polysaccharid der Gruppe C, einem Meningokokken-Polysaccharid der Gruppe W-135, einem von Meningokokken der Gruppe Y und einem von Meningokokken der Gruppe X ausgewählt werden kann.

23. Verfahren nach einem der Ansprüche 2-20, wobei das Polysaccharid aus der Pneumokokken-Gruppe 1, 2, 3, 4, 5, 6A, 6B,7F,8, 9A,9V,9N,10A, 11A,12F,14,15B, 17F, 18C, 19A, 19F, 20, 23F ,33F und 45 ausgewählt werden kann.

24. Verfahren nach einem der Ansprüche 2-23, wobei das Trägerprotein des Polysaccharid-Protein-Konjugats ein Protein ist, das aus der Gruppe TetanusToxoid (TT), Fragment C von TT, Diphtherietoxoid, CRM197, Pneumolysin, Protein D, PhtD, PhtDE.OMPC und N19 ausgewählt wurde.

25. Verfahren nach einem der vorhergehenden Ansprüche, wobei die auf 4-PPY analysierte Probe mindestens ein Konjugat enthält, das aus (a) einem Konjugat aus (i) dem Kapselsaccharid der Serogruppe A von N. meningitidis und (ii) Tetanustoxoid; (b) einem Konjugat aus (i) Kapselsaccharid der Serogruppe C von N. meningitidis und (ii) CRM197; (c) einem Konjugat aus (i) Kapselsaccharid der Serogruppe Y von N. meningitidis und (ii) CRM197; (d) einem Konjugat aus (i) Kapselsaccharid der Serogruppe W135 von N. meningitidis und (ii) CRM197 und (d) einem Konjugat aus (i) Kapselsaccharid der Serogruppe X von N. meningitidis und (ii) Tetanustoxoid ausgewählt wurde.

## Revendications

1. Procédé perfectionné pour tester un échantillon suspecté de contenir de la 4-pyrrolidinopyridine (4-PPY) comprenant les étapes consistant à :
(i) faire incuber un échantillon contenant de la (4-PPY) à une température comprise entre 40°C et 90°C pendant une durée comprise entre 1 h et 3 h
(ii) soumettre un échantillon préchauffé à une chromatographie liquide haute performance en phase inverse
(iii) détecter toute 4-pyrrolidinopyridine (4-PPY).

2. Procédé selon la revendication 1, dans lequel l'échantillon est un conjugué polysaccharide-protéine préparé par chimie de conjugaison par cyanylation en utilisant du tétrafluoroborate de 1-cyano-4-pyrrolidinopyridinium (CPPT).

3. Procédé selon la revendication 1, dans lequel le chauffage au cours de l'étape (i) se fait à une température comprise entre 60°C et 90°C pendant une durée comprise entre 60 minutes et 180 minutes.

4. Procédé selon la revendication 3, dans lequel le chauffage au cours de l'étape (i) se fait à 90°C pendant une durée de 180 minutes.

5. Procédé selon la revendication 1, dans lequel la CLHP en phase inverse se fait en mode isocratique.

6. Procédé selon la revendication 1, dans lequel la colonne d'analyse en phase inverse est à base de silice poreuse en surface et le support de colonne est sélectionné dans un groupe composé de C4, C8, C18, phényle et cyano.

7. Procédé selon la revendication 6, dans lequel la colonne d'analyse est C8 (comme USP L7).

8. Procédé de la revendication 1, dans lequel la phase mobile pour CLHP en phase inverse consiste en un modificateur organique et un acide organique.

9. Procédé de la revendication 8, dans lequel le modificateur organique est sélectionné dans le groupe composé d'acétonitrile et de méthanol.

10. Procédé de la revendication 8, dans lequel l'acide organique est sélectionné dans le groupe composé d'acide trifluoroacétique et d'acide heptafluorobutyrique.

11. Procédé de la revendication 8, dans lequel la phase mobile est composée d'acide trifluoroacétique et d'acétonitrile dans un rapport sélectionné parmi 80:20, 50:50 et 90:10.

12. Procédé de la revendication 11, dans lequel la phase mobile est composée d'acide trifluoroacétique et d'acétonitrile dans un rapport de 80:20.

13. Procédé selon la revendication 12, dans lequel la phase mobile comprend entre 0,01 et 0,1% en volume d'acide trifluoroacétique.

14. Procédé selon la revendication 13, dans lequel la phase mobile comprend entre 0,05% en volume d'acide trifluoroacétique.

15. Procédé selon la revendication 1, dans lequel la phase mobile de CLHP en phase inverse possède un pH compris entre 2 et 2,5.

16. Procédé selon la revendication 1, dans lequel la température de colonne de la phase mobile de CLHP en phase inverse est comprise entre 20°C et 25°C.

17. Procédé selon la revendication 16, dans lequel la température de colonne est de 25°C.

18. Procédé de la revendication 1, dans lequel le débit pour 4-PPY en phase inverse est compris entre 0,8 et 1,2 ml/min.

19. Procédé de la revendication 1, dans lequel la détection est réalisée à une longueur d'onde de 280 nm.

20. Procédé selon la revendication 1, dans lequel la teneur en 4-PPY estimée en soumettant l'échantillon à un chauffage avant la CLHP-PI est au moins 4 fois plus importante que la teneur en 4-PPY obtenue avec une CLHP-PI sans que l'échantillon ait été préchauffé.

21. Procédé de n'importe laquelle des revendications 2-20 dans lequel le polysaccharide capsulaire est un polysaccharide sélectionné parmi un polysaccharide méningococcique, un polysaccharide pneumococcique, un polysaccharide d'haemophilus influenzae de type B, un polysaccharide Vi de salmonella typhi, ou un polysaccharide de streptocoque de groupe B.

22. Procédé de n'importe laquelle des revendications 2-20 dans lequel le polysaccharide peut être sélectionné parmi un polysaccharide méningococcique de groupe A, un polysaccharide méningococcique de groupe C, un polysaccharide méningococcique de groupe W-135, un méningocoque groupe Y et un méningocoque groupe X.

23. Le procédé de n'importe laquelle des revendications 2-20 dans lequel le polysaccharide peut être sélectionné dans le groupe pneumococcique 1, 2, 3, 4, 5, 6A, 6B, 7F, 8, 9A, 9V, 9N, 10A, 11 A, 12F, 14, 15B, 17F, 18C, 19A, 19F, 20, 22F, 23F, 33F et 45.

24. Le procédé de n'importe laquelle des revendications 2-23 dans lequel la protéine de transport des conjugués polysaccharide-protéine est une protéine sélectionnée dans le groupe composé de toxoïde tétanique (TT), fragment C de TT, anatoxine diphtérique, CRM197, pneumolysine, protéine D, PhtD, PhtDE, OMPC et N19.

25. Procédé de n'importe laquelle des revendications précédentes dans lequel l'échantillon qui est testé pour la 4-PPY comprend au moins un conjugué sélectionné parmi (a) un conjugué de (i) le saccharide capsulaire de meningitidis de sérogroupe A N et (ii) toxoïde tétanique ; (b) un conjugué de (i) saccharide capsulaire de meningitidis de sérogroupe C N et (ii) CRM197 ; (c) un conjugué de (i) saccharide capsulaire de meningitidis de sérogroupe Y N et (ii) CRM197 ; (d) un conjugué de (i) saccharide capsulaire de N.meningitidis de sérogroupe W135 et (ii) CRM197 ; et (d) un conjugué de (i) saccharide capsulaire de meningitidis de sérogroupe X N et (ii) toxoïde tétanique.
